(19) Europäisches Patentamt
European Patent Office
Office européen des brevets

(11) **EP 2 501 278 B1**

(12) **EUROPEAN PATENT SPECIFICATION**

(45) Date of publication and mention of the grant of the patent:
**29.09.2021 Bulletin 2021/39**

(21) Application number: **10834944.0**

(22) Date of filing: **18.11.2010**

(51) Int Cl.:
*A61B 5/00* (2006.01)   *A61B 5/11* (2006.01)
*A61B 5/026* (2006.01)   *A61B 5/024* (2006.01)
*A61B 5/029* (2006.01)   *A61B 5/113* (2006.01)

(86) International application number:
**PCT/US2010/057188**

(87) International publication number:
**WO 2011/068687 (09.06.2011 Gazette 2011/23)**

(54) **APPARATUS FOR SENSING BLOOD FLOW AND HEMODYNAMIC PARAMETERS**

VORRICHTUNG ZUR ERFASSUNG EINES BLUTFLUSSES UND HÄMODYNAMISCHER PARAMETER

APPAREIL POUR DÉTECTER LE DÉBIT SANGUIN ET DES PARAMÈTRES HÉMODYNAMIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.08.2010 US 861882**
**24.08.2010 US 861888 P**
**18.11.2009 US 262336 P**
**18.11.2009 US 262331 P**

(43) Date of publication of application:
**26.09.2012 Bulletin 2012/39**

(73) Proprietor: **Texas Instruments Incorporated Dallas TX 75243 (US)**

(72) Inventors:
• **PANDIA, Keya, R.**
**Stanford**
**CA 94305 (US)**
• **RAVINDRAN, Sourabh**
**Dallas**
**TX 75251 (US)**
• **COLE, Edwin, R.**
**Highland Park**
**TX 75219 (US)**

(74) Representative: **Zeller, Andreas et al**
**Texas Instruments Deutschland GmbH**
**Haggertystraße 1**
**85356 Freising (DE)**

(56) References cited:
WO-A2-2006/060205   JP-A- 2000 041 959
JP-A- 2004 305 268   US-A- 4 510 944
US-A- 5 935 081   US-A1- 2004 215 264
US-A1- 2005 038 349   US-B1- 7 139 609
US-B2- 7 186 220

**Description**

This relates to apparatus and methods for measuring, monitoring and analyzing biological and physiological functions of the human body, and to biomedical instrumentation and signal processing relating thereto.

BACKGROUND

The usefulness of hemodynamic data challenges the art to find methods and devices for obtaining, isolating, determining and monitoring such data more simply, economically and more efficiently. Hemodynamics as discussed herein includes the study of blood flow-related data directly or indirectly related to blood flow, such as: heart stroke volume, cardiac output, pre-ejection period, contractility (ability of heart to contract, inotropy), and related causal or caused bodily dynamics such as exercise and exercise recovery, and the Valsalva maneuver (such as when pushing or straining while holding one's breath, or otherwise doing the maneuver in a medical test). Measurement of blood flow, hemodynamics and cardiovascular performance is integral to a full assessment of an individual's health. Specifically, patients with past conditions of heart disease like heart failure (potentially arising out of one or more of many causes like coronary artery disease, heart valve or heart muscle disorders, past myocardial infarction, hypertension etc.) may need constant monitoring in order to improve a person's quality of life via timely and appropriate diagnostic interventions. While the physiological mechanisms underlying these conditions are fairly well understood, the technology to monitor these physiological vitals calls for considerable improvement. Impedance cardiography, doppler echo cardiography, continuous blood pressure monitoring, etc., are believed obtrusive and expensive, and poorly-suited for ambulatory monitoring

Documents US 5935081 A, US 2004/215264 A1, and JP 2004-305268 A disclose the extraction of various heart-related signals from an acceleration signal.

SUMMARY

Generally, and in one form of the invention, an electronic monitoring device includes a triple axis accelerometer sensor having an output, and an electronic processor having an input coupled to the output of the sensor and an output. A memory is coupled to the electronic processor and provides instructions for the electronic processor to isolate a cardiac signal including cardiac pulses combined with other cardiac signal variations. The electronic processor is further operable to execute a filter that separates a varying blood flow signal from the cardiac pulses, and to output information based on at least the varying blood flow signal, wherein the electronic processor is further operable to also separate cardiac pulses from the cardiac signal and to produce an electronic representation of a hemodynamic parameter based on a time difference between a selected cardiac pulse and a peak in the varying blood flow signal after the selected cardiac pulse.

The present invention is defined by the features of claim 1. Further advantageous features are defined in the dependent claims.

BRIEF DESCRIPTION OF THE DRAWINGS

FIG. 1 is a partially-block, partially-pictorial, partially graphical depiction of an inventive structure for separating a blood flow signal from heart and other signals using one or more axes of a single chest accelerometer sensor; and a timemagnified waveform inset is a voltage-versus-time graph of three concurrent waveforms including an ECG signal, a filtered heart signal from the Z-axis sensor, and a blood flow signal filtered from Y-axis of the accelerometer sensor.

FIG. 2 is a block diagram of an inventive wireless system structure.

FIG. 3 is a combined flow diagram of processes not falling under the scope of the present invention for separating a heart signal from body motion and noise using Z-axis accelerometer sensor input and separating a blood flow signal using Y-axis accelerometer sensor input and further electronically processing the heart signal and blood flow signal jointly for hemodynamics.

FIG. 4A is a flow diagram of a process not falling under the scope of the present invention for separating a heart signal from body motion and noise using Z-axis sensor input such as for use in FIG. 3 or with FIG. 4B.

FIG. 4B is a flow diagram of a process not falling under the scope of the present invention for separating a heart signal and blood flow signal from each other in Y-axis sensor input, for hemodynamics and heart rate.

FIG. 5 is a block diagram of an inventive structure for variably combining accelerometer signals from multiple axes in various proportions.

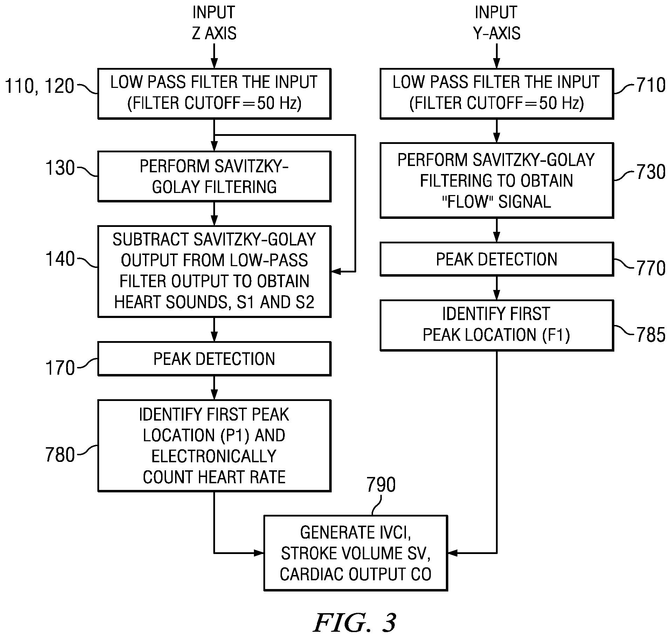

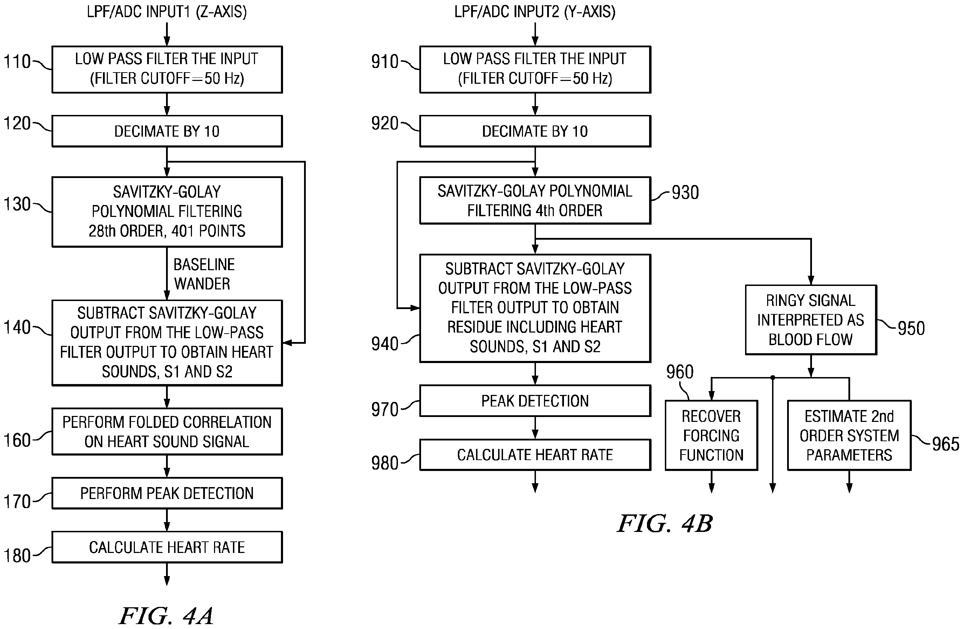

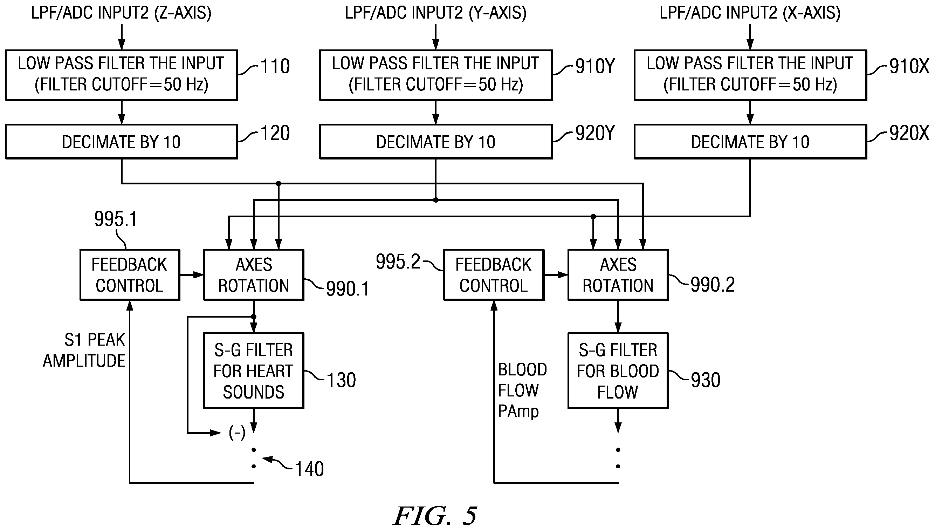

The processes shown in Figs. 3, 4A and 4B illustrate the operation of the device, but do not fall under the scope of the present invention. This also applies to the processes referred to in the context of Fig. 5.

DETAILED DESCRIPTION OF EXAMPLE EMBODIMENTS

Example embodiments are described in an implementation for estimating blood flow and hemodynamic parameters from a single chest-worn sensor. The described embodiments enable measurement of blood flow trends (stroke volume, cardiac output) and other hemodynamic parameters (contractility, pre-ejection period, isovolumic contraction interval) in a non-invasive and minimally obtrusive way. In an advantageous approach, a single miniature sensor (such as a MEMS accelerometer coupled with a data acquisition signal processing embodiment) extracts hemodynamic parameters from an in- plane-of-chest accelerometer Y-axis parallel to a superior-inferior line from head-to-feet.

These benefits are obtained by themselves and with heart pulse detection, respiration detection, motion detection and other features. Ambulatory and other monitoring of cardiac activity including ambulatory hemodynamic monitoring can find widespread applications in home health monitoring of patients with a history of cardiovascular conditions, monitoring older adults, ICU and hospital monitoring, monitoring vital signs in gurneys or rolling patient transfer beds, in mobility aids like scooters and wheelchairs, ambulances, at accident and trauma sites or in a clinic, allied medical testing facility, airport, etc., and for fitness monitoring at exercise centers, stores and elsewhere. Such capability is directly relevant in applications that involve ambulatory monitoring of cardiovascular and cardio-respiratory health. Among advantages of some embodiments, are: a) single sensor and a single signal used to extract several hemodynamic vitals such as any, some or all of changes in stroke volume, changes in cardiac output, heart rate, isovolumic contraction interval, etc.; b) minimal obtrusion (miniature sensor taped on the chest); c) minimal inconvenience in continuous monitoring applications; and d) low cost and convenience with sensors carried by disposable patches and tapes.

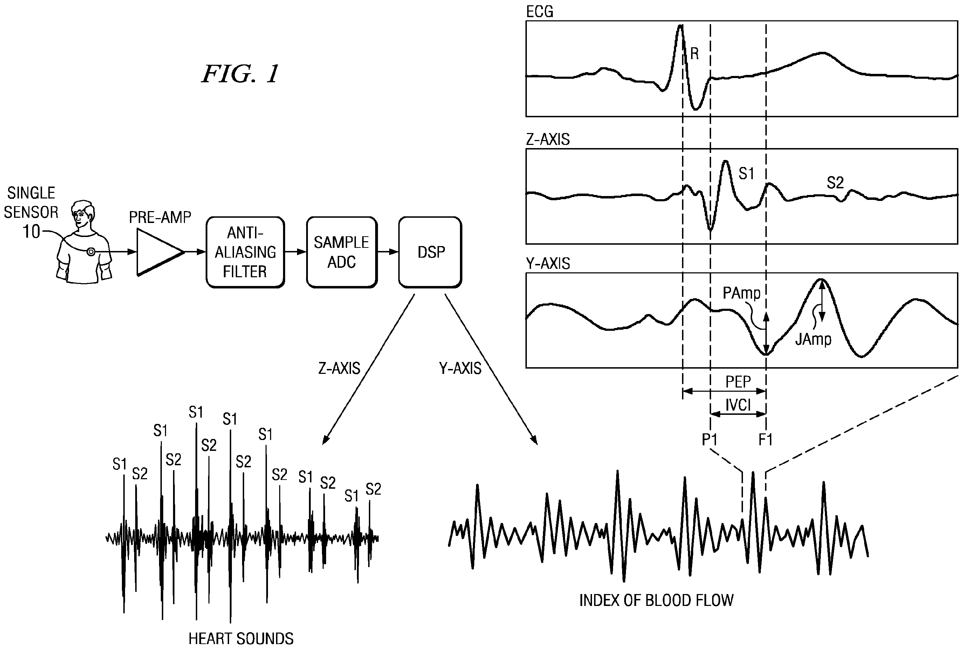

Isovolumic contraction interval (IVCI) is the duration in early systole when the heart ventricles contract with no change in volume. During IVCI, myocardial muscle fibers shorten but have not developed enough pressure in the ventricles to open the aortic and pulmonary valves. IVCI occurs after closure of the mitral and tricuspid valves and before opening of the semi-lunar valves. IVCI is electronically measured or estimated as the time difference between S1 peak P1 and beginning time F1 of first peak of the vertical Y-axis accelerometer blood flow signal. ICVI is expected to correlate well with the time difference F1-P1 (see FIG. 1 inset).

Stroke volume (SV) is the difference between the end diastolic volume and end systolic volume and is a measure of the blood pumped by the heart per cardiac cycle. A conventional pulse contour method calculates a blood flow variable (milliliters/sec) from the pressure signal and computes the stroke volume by integrating the blood flow signal over a cardiac period. By embodiments of structure herein, a peak amplitude PAmp and Jamp of the flow signal derived by filtering from the accelerometer sensor is used to compute relative changes in SV. SV is computed by first applying a blood pressure signal, or a signal related thereto, to a model of the arterial system. One such model is a Windkessel model, non-linear to model behavior of the arteries as they expand under blood pressure, which regards the blood pressure as analogous to a voltage applied to a series-parallel network having a series impedance to an output, and a parallel resistor-capacitor combination across the output. Blood flow is analogous to the voltage across the output of the circuit. The integrated output voltage over a period of heart rate S1-to-S1 is related to stroke volume for that period and is repeatedly computed. Some other models analogize a reflective electrical transmission line to the arterial system. Any model appropriate to the purposes at hand is employed.

Cardiac output (CO) is the volume of blood pumped by the heart per minute, and defined as the product of stroke volume and heart rate (HR).

Pre-ejection period (PEP) is the time interval between onset of ECG QRS complex and the cardiac ejection. PEP is calculated as R-F1 interval from the beginning of the ECG QRS complex to the beginning of the first peak in the accelerometer signal (see FIG. 1 inset).

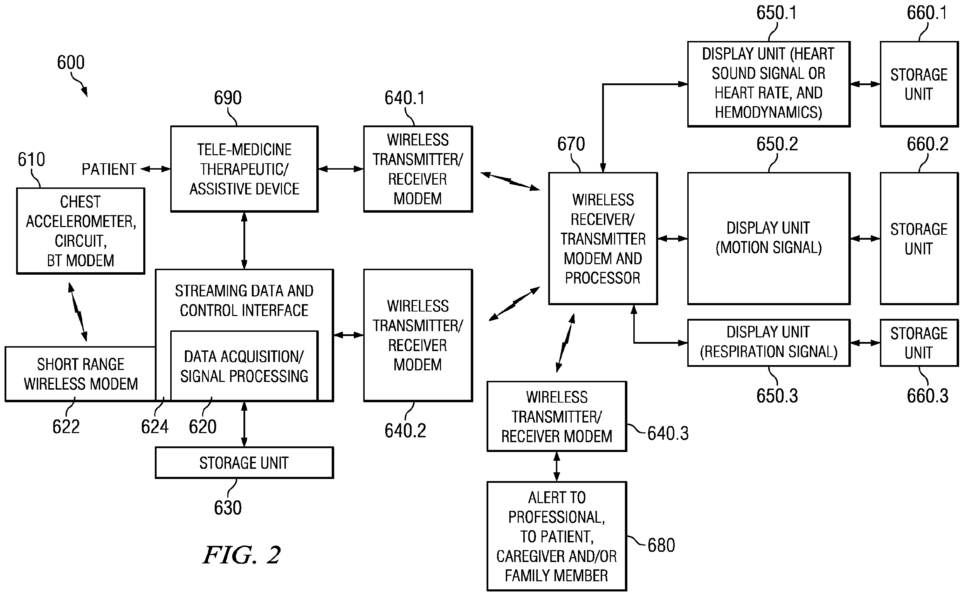

The electronic measurement in one example jointly processes the flow signal from filtering the accelerometer-derived Y-axis (FIG. 1); and the simultaneous ECG as shown in FIG. 2 or estimating from IVCI as described elsewhere herein.

Ventricular contractility (VC) measures intrinsic ability of the heart to contract and can be estimated from stroke volume (SV). Increase in stroke volume causes an increase in contractility. Contractility (VC) is measured herein by trending the pre-ejection period PEP or estimating from IVCI as described elsewhere herein.

FIG. 1 illustrates a system embodiment that provides a convenient monitoring device and measurement set-up. A miniature (weight = 0.08 gram, size = 5x5x1.6 mm) triple axis, low-power, analog output MEMS accelerometer (e.g., STMicroelectronics Part No. LIS3L02AL) is taped onto the chest (e.g., a few inches to the left of the sternum along the third or fourth rib). (Taping the accelerometer sensor or using a chest band presses the accelerometer sensor to or against a bare or shaved portion of the chest and efficiently couples chest acceleration to the sensor.) An acceleration signal along the Z-axis -- the dorso-ventral direction orthogonal to the plane of the chest -- corresponding to the heart sounds is captured. Simultaneously, the acceleration signal along the Y-axisin the plane of the chest and oriented vertically upwards -- corresponding to the blood flow and related hemodynamics is also captured by the same MEMS (microelectromechanical system) accelerometer. In this way, the acceleration signal along the in-plane vertical axis (Y-axis) is also captured. The accelerometer may have a broadside portion such that two perpendicular axes of acceleration sensitivity are parallel to the broadside portion.

The chest acceleration signals from both axes are, for instance, concurrently AC coupled (high pass roll-off was dropped about 10x in an example compared to a non-critical 3 Hz) and separately and in parallel are amplified with a gain of 100 and low pass filtered - for anti-aliasing - through a three-stage, five-pole Sallen-and-Key Butterworth filters with a 1 kHz corner frequency. Two commercial quad operational amplifier packages (e.g., Linear Technology Part No. LT1014CN) are used for the analog frontend. The accelerometer signals are then each sampled at 10,000 samples/sec using a data acquisition card (e.g., as available from National Instruments, Austin, TX) and captured and stored on a computer using MATLAB software (Version 2007b, The Mathworks, Natick, MA).

The AC coupling with approximately 3 Hz cutoff, which is a non-critical roll-off frequency, is provided, for example, by a series coupling capacitor C coupled to an input resistance established for the amplifier.

In an example for detection of primary heart sounds and cardiac activity (illustrated by FIG. 3 (left side) and FIG. 4A), the acceleration signal is digitally low pass filtered in a step 110 at 50 Hz (using, e.g., a 3326 tap digital FIR filter with a steep 80 dB roll-off over 20 Hz) and decimated in a step 120 by a factor of 10. Roll-off frequency less than 60 Hz attenuates 60 cycle USA power line interference with biomedical signals of interest, and roll-off may be made less than 50 Hz for applicable countries using 50 Hz. While the roll-off frequency could be made higher, this finite impulse response (FIR) filter also desirably attenuates white noise above the frequency range of the signals being monitored. Also in a Phase 1, a high order Savitzky-Golay (S-G) polynomial smoothing filter 130, using 28th order and 401 point frame, is used to capture the relatively slow-varying motion wander, and leave out the more rapidly varying heart sound signal components. (MATLAB syntax for such filter is g = sgolayfilt(X,28,401), where g is the filter output and X is a latest input column vector of 401 sample values of windowed data.) In a Phase 2, the smoothing filter 130 output is subtracted in a step 140 from the decimated low pass filter (LPF) output to obtain heart sounds S1 and S2. A folded correlation process in a step 160 then enhances and strengthens the polynomial filtered S1/S2 peaks in the motion-removed acceleration signal. Then the location of the peaks is threshold-detected in a step 170 using an electronic amplitude-based peak picking process, and the selected peaks are counted in a step 180 to calculate heart rate (HR).

A reference electrocardiogram (ECG or EKG) is acquired simultaneously in a three-electrode (single lead) ECG amplifier by analogous signal path for system testing to compare with the accelerometer-derived cardiac signal and independently extract, e.g., heart pulses, pre-ejection period (PEP), contractility (VC).

The system extracts primary heart sounds (S1 and S2 produced by the heart valve pairs closing at the ends of the diastolic and systolic periods respectively of the cardiac cycle) using a Z-axis sensor of the accelerometer worn on the chest (see FIG. 3 left side and Appendix). (The term "heart sound" refers in an expansive way to a signal analogous to cardiac S1, S2, and/or heart murmur or other cardiac waveform features, obtained from the processing of accelerometer data or other sensor data, and not necessarily to an audible sound.) Heart sound S1 is concurrent with tricuspid and mitral valve activity and shows on a seismocardiogram as a pulse bundle. Heart sound S2 is mainly associated with pulmonary valve and aortic valve activity.

Hemodynamics from Y-axis: Striking differences appear between the signals picked up by the Y-axis (in the chest plane) and the Z-axis. S1 and S2 align well in shape and timing in both axes, but the additional features (lower in frequency with the biggest peak between S1 and S2) occur very strongly along the Y-axis, but are hardly present in the Z-axis direction, as of a blood flow-related signal. The phrase flow signal or flow-related signal or blood flow signal is a useful identifying label for this chest acceleration component, recognizing that the accelerometer is sensing a component of chest acceleration in meters/sec$^2$. This time-varying acceleration component appears to be a body-reaction acceleration or skin-shear acceleration approximately parallel to the superior-inferior body axis in response to blood flow and is related to an overall force of cardiac contraction in newtons and/or to systolic blood pressure in newtons/square meter and/or to blood flow acceleration in milliliters/sec$^2$ and/or blood flow velocity in milliliters/ sec. Dynamics of the blood varies spatially and with time in the interior and along the blood vessels of the arterial system emanating from the heart. Arterial wall friction and elasticity are involved, and these change if hardening of the arteries occurs. Accelerometer signal(s) are accordingly processed as for FIGS. 3-5 and post-processed and interpreted, e.g., using a system of FIG. 2 to fully realize benefits.

FIG. 2 shows a wireless system embodiment 600 for a hemodynamic, cardiac, respiration and motion monitoring system with remarkable component embodiments. (Some system embodiments can be wired embodiments.) Accelerometer sensor 610 and its electronic circuit also have a Bluetooth or other short range wireless modem wirelessly coupled to another short range wireless modem 622 coupled via a streaming data and control interface 624 to a data acquisition signal processing unit 620. Further, modems 640.2 and 670 provide wireless transmission and reception so data acquisition signal processing unit 620 communicates via its modem 640.2 to the remote wireless transceiver unit or modem 670 that is coupled to be one or more display units 650.i and their storage unit(s) 660.i. In this way, telemedicine applications are supported. Acquisition signal processing unit 620 and its modem 640.2 are suitably provided in a residence or ambulance or on the person or in a wheelchair or gurney. The wireless transceiver 670 and display unit(s) 650.i are suitably provided in a clinic, hospital, medical monitoring center or otherwise. Either or both ends of the wireless system may be mobile, e.g., a modem 640.3 and alert/processor/display 680 when a professional in a vehicle is urgently needed to review data in case of emergency and to respond with instructions.

In FIG. 2, devices provide automatic cautionary responses, warnings, and/or automated monitored therapeutic responses. Upon occurrence of undue excursions of one or more measured parameters or relationships among parameters detected by signal processing unit 620, the remote processor 670 alerts any one or more of medical professional, patient, caregiver, and/or family member via a modem 640.3 and alert/processor/display unit 680 by sending an acknowledgable voice call or message and/or written alert or e-mail. Another modem unit 640.1 couples to a telemedicine therapeutic or assistive device 690 for assisting the patient in some pharmacological, informational, or physically assistive way by administering a medicine, or adjusting a dosage or otherwise. In case of excursions that indicate an extreme medical emergency, the data acquisition signal processing unit 620 may be permitted to locally control the therapeutic or assistive device 690 temporarily and in a maximally-safe way until remote commands are received or first responders can arrive. Mere removal or inadvertent accelerometer 610 detachment from chest is distinguished by electronic processing 620 from affirmatively detected excursions. Telecommunications care (tele-care) assistance may be rendered responsive to sensor 620 data by activating motorized apparatus 690 such as to adjust a motorized bed, or move a scooter into proximity for patient use, or servo-mechanically actuate and flush a toilet unit, or open a tub drain to empty a tub, or some other assistance.

Types of embodiments, among others, can form: 1) a patient-worn medicalsensor and/or therapeutic device that is wired or has a wireless modem; or 2) a patient-worn signal processing and modem module on a belt clip that connects or wirelessly couples to such a sensor and wirelessly couples to a premises gateway or directly transmits to a remote location; or 3) a sensor, signal processor, memory, and modem together in a micro-miniature device that is taped to the chest and communicates to a router or gateway locally, and the latter communicates remotely; or 4) a local router or gateway that includes signal processor, memory, and multiple types of modem to communicate with the sensor and separately communicate remotely, such as in a patient home-based system to telecommunicate to clinic or hospital; or 5) a complete medical clinic system.

Peripherals and interfaces can include a UART (universal asynchronous receiver/transmitter) data interface and MCSI (multi-channel serial interface) voice and data wireless interface for an off-chip IEEE 802.15 (Bluetooth and low and high rate piconet, Zigbee, and personal network communications) for a wireless circuit. The Bluetooth or Zigbee wireless interface is useful for receiving from and controlling the accelerometer sensor 10 (610) and its associated analog circuitry and digital to analog-to-digital converter ADC in FIG. 1. In arrangements including ECG electrodes and/or a chest microphone, the analog circuitry at the taped-on sensor unit also includes couplings from such pickup elements to the Bluetooth or Zigbee short distance transceiver from the chest sensor.

Turning to FIG. 3 (right side) and FIG. 4B, the Y-axis signal is filtered or smoothed using a low order (4th order) Savitzky-Golay polynomial filter with a window size roughly 200 milliseconds. Different window length and polynomial orders are feasible. Also, specific polynomials and orders are illustrative and not limiting because they are related to the signal and the sampling rate. The smoothing filter extracts the slow varying (lower frequency) blood flow signal separated from the residue of the heart sounds (S1 and S2). Using Savitzky-Golay (S-G) filtering as the smoothing filter is one of various possible ways of extracting the flow signal from the Y-axis. Slow varying respiration and body motion baseline wander (e.g., below about 0.5Hz) is also removed or separated from the blood flow signal in some embodiments by either cascading or combining a high pass filter to attenuate the respiration wander, or using a smoothing filter to isolate the respiration wander and subtracting to yield a residue signal. Further in some embodiments, the respiration is separated from body motion either by detection of variations of S1-S1 interval or by monitoring amplitude modulation on the S1 peaks.

An extracted flow signal from filtering of the raw Y-axis signal is called a blood flow component, or flow signal, herein. A 200 msec. window size nicely encompasses either straight or curved portions within a cycle of the oscillating blood flow signal as the raw Y axis signal stream progresses through the filter window. A primary "flow" peak (between S1 and S2) from the accelerometer-based waveform is prominent on the blood flow signal and bears some relationship to a signal from a ballistocardiogram (BCG) while not being identical to a BCG. This flow signal appears related to acceleration of surge blood as pushed out of left ventricle into aorta. The flow signal has noticeable ringing to it, and can convey some useful information about heart and valve mechanics and possibly other subjects, as described further elsewhere herein. Such information is suitably generated and communicated electronically in the wired or wireless apparatus of FIG. 2 operating according to processes described in FIGS. 3 and FIG. 4A, and/or in FIG. 4B.

In FIG. 3, a process is represented physically in system storage unit or memory of FIG. 2 and executed on the signal processing unit of FIG. 2 or digital signal processor DSP of FIG. 1. In FIG. 3 (left side), Z-axis signal processing 110-170 occurs as described in Appendix or elsewhere herein, and additionally the peak detection 170 of the S1pulse is followed in FIG. 3 by a time determination 780 of a first peak location in time P1 of the first heart sound peak S1 in each heart beat. Counter operation in response to the S1 pulses (and S2 pulses or both) electronically generates a heart rate signal as in FIG. 4A.

In FIG. 3 (right), Y-axis signal processing at step 710 low pass filters (LPFs) the Y-axis input (filter cutoff 50 Hz) and then a step 730 S-G polynomial-filters (or otherwise filters effectively) the LPF signal from step 710 to produce a flow signal output from step 730. A step 770 performs electronic peak (and trough) detection on the flow signal to identify a flow peak amplitude PAmp. A succeeding step 785 identifies the location F1 of that peak in time, as illustrated graphically in FIG. 1 inset. Then, in FIG. 3, both the heart rate signal output HR and first peak location in time PI from step 780 are combined with and/or compared with first flow signal peak time location F1 from step 785 for each heartbeat. The time difference of F1-P1 represents or is proportional to the isovolumic contraction interval IVCI. IVCI in FIGS. 3 (and FIG. 1) is estimated, for example, as the time difference between time PI of the peak of the S1 waveform (derived from the residue signal based on the Z-axis sensor) and the time F1 of the first peak of the flow signal derived from the Y-axis sensor. (In FIG. 4B, some embodiments determine heart sound time PI based on Y-axis processing instead of Z-axis processing.)

In FIG. 3, Stroke volume (SV) and relative changes therein are computed at a step 790 proportional to the peak amplitude PAmp of the flow signal derived by filtering from the Y-axis accelerometer sensor. SV in some embodiments is computed proportional to the peak amplitude PAmp of the flow signal multiplied by the ICVI estimate. Cardiac output (CO) in FIG. 3 is electronically generated by multiplying stroke volume SV by heart rate (HR) derived from Z-axis processing from the step 140 residue from accelerometer sensor filtering and the result is supplied to display 550.i or 650.i and other devices as discussed in connection with FIG. 2. In FIG. 3, hemodynamic parameters including all of IVCI, SV, CO, PEP, and VC and others are obtained as described herein.

The inset to FIG. 1 depicts an ECG, heart sound signal, and flow signal from the accelerometer 610. The time PI of occurrence of the S1 sound and the peak location in time F1 of the flow signal in FIG. 1 inset are used to provide a time difference for electronically estimating isovolumic contraction interval ICVI. Pre-ejection period PEP is electronically derived using the time difference between the peak of ECG signal and peak F1 of the flow signal. A peak amplitude of the flow signal is signified by PAmp in FIG. 1 inset. Peak amplitude PAmp is electronically derived as the difference between the highest peak of the flow signal during a given heartbeat and a baseline average of the flow signal as indicated by a medial dotted line for that heartbeat. A succeeding peak Jamp is also depicted. An electronic process generates signals representing a value of PEP for every heartbeat and a value of PAmp for every heartbeat.

Superimposed flow signal ensembles for individual heart beats aligned to the ECG R-waves show relative changes (up to 2x) in signal amplitude and timing (jitter/dilation) during recovery of subject from mild exercise. Some embodiments also generate a signal representing the variance of the jitter in either or both of ICVI and PEP during a period of exercise and/or during a period of rest, as well as changes in such variance value, as an indication of heart function and changes therein. In some embodiments that measure pre-ejection period (PEP) and contractility (VC), one of the ECG electrodes is physically combined or associated with the accelerometer sensor of FIG. 1. A miniature microphone can be provided along with the accelerometer in the same chest-worn physical unit 10 for obtaining heart sound audio for parallel processing. Concurrent signals from a single chest accelerometer can be separated to get physiologically relevant information: heart rate, activity/motion, respiration and intrapleural or intra-thoracic pressure changes, hemodynamics (timings and amplitudes and changes), cough, sneeze, snore, speech, breathing sounds, gait, respiration and other activities.

Some accelerometer sensor embodiments may be called upon to estimate Pre-ejection period (PEP) and contractility (VC) or trends therein, but lack the ECG electrodes, such at a residence. IVCI is based on S1-to-F1 interval in FIG. 1 inset. IVCI is guardedly either used as a proxy directly or for trends for pre-ejection period (PEP) (and for use in obtaining contractility (VC)) where measurements indicate that it is sufficiently-correlated to PEP or trend therein, or at least sufficient for monitoring variations in PEP and VC to detect excursions from expected parameter ranges needing a clinic visit. Previous clinical measurements with both accelerometer and ECG calibrate a time interval adjustment value $\alpha$ and scale value $\beta$ to estimate PEP from IVCI according to Equation (15), where measurements indicate that adjustment $\alpha$ and scale value $\beta$ are statistically significant and sufficiently accurate, and may be measured and downloaded as a table of values $\alpha$ (HR, etc.) and $\beta$ (HR, etc.) to estimate PEP from IVCI adjusted for heart rate (HR, etc.). Such approach is likely acceptable only when no other alternative is immediately available at the remote location.

$$PEP = \beta * IVCI + \alpha \qquad\qquad (15)$$

Some embodiments post-process the peak amplitude PAmp on Z-axis or other axis signal amplitude (which correlates well with SV and CO) to provide or derive time-varying output signals and displays in FIG. 2. Such signals and displays show estimation for hemodynamic parameters such as SV and CO and others derivable directly at FIG. 3 step 790 from the amplitude/power of the cardiac S1 pulse either independently of, or in combination with, information from the blood flow signal. The estimation may differ from SV and CO themselves by an additive constant and a scale factor, for monitoring applications that begin with a pre-existing physiological state and are interested in subsequent variations and/or unusual departures. Notice that the SV and CO hemodynamic parameters vary much more slowly with time t (e.g., less than 0.2 Hz or less than 0.1 Hz or so) than respiratory variation in the peak amplitude signal PAmp(t), so that SV and CO and respiration can be derived from or filtered out of the peak amplitude signal PAmp(t) and provided for display and recording.

In FIGS. 4A and 4B, a process is represented physically in system storage unit 630 or memory of FIG. 2 and executed on the signal processing unit 620 of FIG. 2 or digital signal processor DSP of FIG. 1. In FIG. 4A, Z-axis signal processing 110-180 is generally analogous to Z-axis processing of FIG. 3 (left side), and outputs heart rate at step 180 and need not be further detailed. In FIG. 4B, Y-axis signal processing 910-940, 970, 980 independently also derives heart rate by obtaining a residue signal from the Y-axis input with the S-G filtered signal subtracted out. Electronic peak detection of the residue signal at step 940 is followed by peak detection 970 and counting 980. The heart rate signal output from step 980 is either combined with and/or compared with the heart rate output of FIG. 4A or used instead of and without the heart rate output of FIG. 4A, depending on embodiment.

Further in FIG. 4B, the S-G filtered signal from step 930 itself is a ringy flow signal interpreted as blood flow and provided as an electronic output 950 for display 650.i and optional storage 660.i. Also, flow signal 950 is processed at step 960 to recover a Forcing Function F(t) electronic output indicative of cardiac function. That flow signal is processed at a step 965 to estimate one, two, or all of a triplet of $2^{nd}$ order model parameters for mass m, dashpot $\rho$ (rho), spring $\gamma$ (gamma) as further electronic outputs. FIG. 3 steps 770, 785 and 790 in FIG. 4B use the flow signal from step 930. The outputs can be further post-processed into electronically-represented interpretations and displays in FIG. 2 of internals of chest and heart and states of function. Post-processing is applied to the damped oscillatory flow signal at 950 of FIG. 4B (derived from the Y-axis accelerometer sensor) as from a 2nd order system model that has a forcing function F(t) (newtons) and constant coefficient parameters for mass m, dashpot $\rho$ (rho), spring $\gamma$ (gamma) in its 2nd order linear differential equation of Equation (16). Variable y represents physical displacement of chest sensor from average y position (conceptually measured from some stationary point of reference on the body, such as the hips, relative to which the chest is displaced).

$$m\,\partial^2 y/\partial t^2 + \rho\,\partial y/\partial t + \gamma\,y(t) = F(t) \qquad\qquad (16)$$

A model of a standing individual has a first triplet of those parameters subscripted "1"; or lying prone has a second triplet subscripted "2." In standing and prone positions, Y-axis sensor is positioned same on chest, parallel to superior-inferior axis of body symmetry. Flow signal 950, g(i) derived by step 930 from Y-axis sensor (e.g., by S-G filtering), is a series of samples g(t) each substantially proportional to the second derivative $\partial^2 x/\partial t^2$ itself in Equation (16). Dashpot parameter $\rho$ introduces energy dissipation, and time constant $\tau$ of decay of the damped oscillatory signal is related to mass parameter m (kilograms) divided by the dashpot parameter $\rho$ (newtons/(meters/sec)).

$$\tau = m/\rho \qquad\qquad (17)$$

Frequency $f_s$ of the damped signal is related to $(1/2\pi)\sqrt{(\gamma/m)}$, i.e., the square root of the ratio of the spring parameter $\gamma$ (newtons/meter) divided by mass parameter m (kilograms):

$$f_s = (1/2\pi)\sqrt{(\gamma/m)} \qquad\qquad (18)$$

Post-processing suitably estimates F(t) /m , as by numerical integrations from damped oscillatory flow signal waveform from y-axis, S(t) = $\partial^2 y/\partial t^2$ using Equation (16) written as Equation (19). The numerical integration begins as each spindle-shaped accelerometer Y-axis waveform commences in FIG. 1 (3rd waveform) for a given heartbeat. Constants of integration are zero position, zero velocity. For applications based on the shape or morphology of the forcing function F(t), the mass m is merely a constant of proportionality that does not affect the shape, or mass can taken as that of head and torso, a fraction (e.g., 0.6) of body mass in kilograms. Time constant $\tau$ is numerically estimated as the length of time from peak of the spindle-shaped acceleration waveform to the time when the waveform is about 2/3 dissipated (i.e., reduced on later end of the spindle to 1/e of its earlier peak amplitude, where e is the base of natural logarithms 2.71828...). Time constant $\tau$ is about a quarter of a second. The frequency $f_s$ is numerically estimated as the number of cycles in some portion of the spindle-shaped acceleration waveform divided by the time in seconds occupied by that portion. In FIG. 1, the frequency $f_s$ is about 8 Hz.

$$F(t)/m = S(t) + (1/\tau) \int_0^t S(t)dt + (2\pi f_s)^2 \int_0^t \int_0^t S(t)dt \qquad (19)$$

Forcing function $F_Y$ (t) component parallel to the Y-axis sensor may arise by 1) physical acceleration of the heart upon ventricular contraction and 2) the acceleration of blood expelled from the left ventricle surging into the aorta. Parameter $\gamma$ for springconstant and parameter $\rho$ for dashpot relate to some gross average of mechanical properties of the interiors of chest and abdomen. Mass parameter m probably is related or proportional to mass of torso and perhaps head, but probably not to mass of legs because legs not accelerated in the Y-axis direction. S1-S1 waveform also has a rising amplitude of oscillation immediately preceding the damped oscillation, see FIG. 1, that can be due to entry of blood from venae cavae into right atrium, and right ventricular contraction into the pulmonary artery. Embodiments for extraction and analysis of forcing function $F_Y(t)$ can provide information on cardiac and pulmonary function and hemodynamic data.

X-axis sensor information can be monitored as well as Y-axis and Z-axis. An X-axis sensor is oriented to sense acceleration laterally across the chest. A transverse displacement variable x for purposes of Equation (20-X) represents side-to-side physical displacement of the chest sensor from an average x position (or conceptually also from a point of reference such as the center of mass of the heart relative to which the chest is displaced.) Signal from X-axis sensor is filtered in parallel with and analogous to filtering of Y-axis signal. Because of the assymetrical location and slantwise inclination and of the heart in the chest, the filtered X-axis sensor provides a lateral (side-to-side) component $F_X(t)$ of vector forcing function F(t). Forcing function components $F_Y(t)$ and $F_X(t)$ can provide further useful information on cardiac function, pulmonary function, properties of the pleura, pleural cavity, and pericardium, as well as hemodynamics information relating to the aorta, venae cavae, and pulmonary arteries and pulmonary veins by any suitable process. The parameter triplets are respectively subscripted "1Y" and "IX" to designate a standing position ("1") and the Y-axis or X-axis sensor involved. If the prone position is involved then the subscript "1" is changed to "2."

$$m_{1Y}\, \partial^2 y/\partial t^2 + \rho_{1Y}\partial y/\partial t + \gamma_{1Y}\, y(t) = F_{1Y}(t)\,; \qquad (20\text{-}Y)$$

$$m_{1X}\, \partial^2 x/\partial t^2 + \rho_{1X}\, \partial x/\partial t + \gamma_{1X}\, x(t) = F_{1X}(t). \qquad (20\text{-}X)$$

Turning to FIG. 5, in some embodiments a coin-sized accelerometer sensor 10 and transmitter, transceiver, or transponder chip are light in weight and firmly mounted on a thin, resilient plastic support plate with round smoothed or flanged periphery taped to chest. Accelerometer sensor Z-axis is perpendicular to the plastic support. The chip may harvest power from an interrogation signal, or get power from a small battery also on the plastic support. An MSP430™ processor from Texas Instruments or other low power processor and short distance wireless transmitter can be included and may also have a wireline interface and USB (universal serial bus) connector for instance.

In FIG. 5, the orientation of the X, Y, and Z axes of the accelerometer sensor on the chest may vary, but merely distributes the overall physical acceleration vector a to the different sensor axes according to their vector components. Accordingly, an electronic processing module 990.i for virtual re-orientation or optimization of the accelerometer sensor signals rotates axes by multiplication by a rotation matrix that combines the signals for X, Y, Z as if the accelerometer axes themselves were rotated, as shown by Equation (21). Angle $\theta$ is an angle by which Y-axis sensor is virtually rotated from its affixed position on the chest to align with the foot-to-head direction on the body. An angle $\varphi$ is an angle to virtually rotate Z-axis sensor from its affixed position approximately perpendicular to the chest toward that foot-to-head direction. A vector V represents the Z-axis signal, the Y-axis signal and the X-axis signal and is matrix multiplied electronically in FIG. 5 according to the rotation product R*V, using rotation matrix R expressed by Equation (21).

$$R = \begin{bmatrix} \cos\varphi & (\sin\varphi\,\sin\theta) & (\sin\varphi\,\cos\theta) \\ 0 & \cos\theta & -\sin\theta \\ -\sin\varphi & (\cos\varphi\,\sin\theta) & (\cos\varphi\,\cos\theta) \end{bmatrix} \qquad (21)$$

The axis rotations are suitably customized to maximize signal output (e.g., blood flow, heart sounds). The angles $\theta$ and $\varphi$ are each varied by a respective given feedback control circuit 995.i to maximize the desired type of signal output. In FIG. 5, for instance, some embodiments execute a feedback control 995.1 to thus maximize the heart sound signal for the heart monitoring path, and the axes-rotation parameters for the rotation process 990.1 for a feedback loop 990.1, 130, 140,..., 995.1 established either as a configuration routine before run-time, or dynamically at run-time. The feedback loop rotates the axes to deliver a linear combination of Z-axis and Y-axis (and X-axis can also be useful) as an input in place of the raw Z-axis signal in FIGS. 3 and 4A to the Savitzky-Golay polynomial filter 130 for the Z-axis to maximize the amplitude of the S1 peaks at the output of the Folded Correlation, for heart sound and heart rate monitoring purposes. Analogously, in FIG. 5, some embodiments additionally or alternatively execute a feedback loop 990.2, 930,..., 995.2 by independently rotating axes to deliver a linear combination of X-axis and Y-axis (and Z-axis can also be useful) as an input in place of the raw Y-axis signal in FIGS. 3 and 4B to the Savitzky-Golay polynomial filter 930 for the Y-axis, to maximize the blood flow signal peak amplitude PAmp of FIG. 1. One rotation 990 and one feedback control 995 can operate from signals jointly, like heart sounds amplitude and/or blood flow signal amplitude. Modes of operation and configuration can be activated or disabled by one or more control registers with bits or bit fields for them. A manual mode, if activated, overrides feedback controls and lets a clinician manually optimize virtual rotations of signals on the computer display of FIG. 2.

FILTERING FOR SIGNAL SMOOTHING

In FIG. 3 (left side) and FIGS. 4A and 4B, gross motion is tracked and canceled out from the primary accelerometer-based signal to leave behind what is called a residue signal. A polynomial smoothing filter 130 (for example, a Savitzky-Golay filter) electronically and digitally smoothes a given accelerometer-based data signal stream by least-squares approximation within a specified data window by a polynomial of a specified order, and preserves higher order moments around inflection points or at extrema like peaks and troughs that a digital moving average or low-pass filter does not. The residue signal provides primarily the heart sounds and other cardiac activity. Order of about 20th - 30th order is satisfactory; but lower orders and even a 1st-order may work for some window sizes and applications. Also, low order polynomial filtering example obtains a blood flow signal for hemodynamic monitoring herein. Using a number of points at least approximately half again (1.5 or more times) an order of the polynomial and even substantially higher than that can help to reduce noise.

The smoothing filter 130 of FIGS. 3 and 4A or 730 of FIG. 4B is configured for an order M and frame size (number of sample points N). For a fixed window length, $N_W$ in points, a higher order polynomial will fit high frequency components of streaming data better. For a given order M, a shorter window $t_W$ of time will allow fitting the high frequency component better. One way of optimization estimates the inherent order of the low-frequency component and picks the smallest window that satisfies $N_W > M+1$ and Nw is odd (i.e., Nw = 2N+1). Smaller window size Nw means a smaller number of taps of the multiply-accumulate filter process to do the smoothing filtering. For an accelerometer signal in some applications, order M=1 and window size Nw =3 (sampling frequency is 1000 Hz). In some examples herein, higher orders M and window widths $N_W$ are shown.

In FIGS. 3 (left side) and 4A, motion, heart sounds and heart rate are electronically separated and ascertained from accelerometer 210 data using the following steps: a) low-pass filtering 110 and decimating 120 the accelerometer data; b) Savitzky-Golay filtering 130 to fit the relatively lower frequency motion data; c) subtracting 140 the output of the Savitzky-Golay filter from the low-pass filtered accelerometer data (from step a) to obtain the heart sounds; d) performing 160 folded correlation to enhance the primary heart sounds (S1 and S2) peak locations; and e) peak picking 170 to count the number of S1 peaks in a predetermined or configured segment (time interval) and counting 180 the heart rate HR in beats per minute BPM.

Decimation passes one sample in every $n_D$ samples (not necessarily removing all but 1 sample in exactly ten). Thus, if a sample/ADC delivers $f_S$ samples per second, then a decimation frequency is $f_S/n_D$ samples per second. If a window period is $t_W$ seconds, then window points Nw = 2N+1 is $N_W = 1+f_S t_W/n_D$. Select window period $t_W$ in view of time period of particular features and behavior of interest to be obtained. Select sampling frequency $f_S$ in view of cost, physical size and complexity of FIG. 1 anti-aliasing filter AAF at $0.5f_S$ or less, ahead of sampling $f_S$). Set sampling frequency $f_S$ greater than the Nyquist frequency for sampling the AAF output. Select decimation parameter $n_D$, to yield $f_S/n_D$ higher than 50 Hz LPF after sampling/ADC for effective LPF. Select decimation parameter $n_D$ also to yield a number Nw of window points high relative to filter order M to keep filter noise low while $N_W$ window points are still low in number but enough so filter computations still satisfactorily filter the signal stream in the window. Filter computations are related to the product of the number Nw of points per window multiplied by a rate number $r_W$ of windows processed per second. If $r_W = N_W/t_W$, the computations are proportional to $N_W^2/t_W$, which may motivate fewer window points and longer window times for energy-saving and lower cost. Remarkably, the examples herein satisfy these considerations for some applications and other examples may be devised.

Processes for various forms of step 130, 730, 930 can be partitioned into offline and real-time online electronic processes. The expression $||(x-Ab)||$ in Equation (1) below signifies the sum of squared differences between the [(2N+1)x1] respective data stream vector sample points or stream components and the (2N+1) respective estimates of those stream components provided by multiplying a [(2N+1)xM] transform matrix A times a [Mx1] vector of transform coefficients $b_j$. The number of transform coefficients $b_j$ is M, and they form a [Mx1] vector b. A gradient v is the [Mx1] vector of first partial derivatives with respect to the transform coefficients $b_j$. The number M of coefficients $b_j$ is called the order, and if the number of transform coefficients $b_j$ is M, then the order of the process is M. The [MxM] matrix of second partial derivatives with respect to the transform coefficients $b_j$ is signified by $\nabla \nabla$. The filter procedure involves, and in effect forms, a coefficients change coefficient vector $\Delta b$ for updating an initial transform coefficient estimate b=0 (i.e., all coefficients initialized to zero). This procedure pre-multiplies the matrix of second partial derivatives times the negative of the gradient to obtain that transform coefficients change vector:

$$\Delta b = - (\nabla \nabla \|(X\text{-}Ab)\|)^{-1} \ \nabla \ \|(X\text{-}Ab)\| \ . \qquad (1)$$

Since the Equation (1) involves a quadratic expression and starts from b=0, the process directly finds the values of the transform coefficients $b = \Delta b$ in one pass without iterating additionally.

Equation (2) represents the result of performing the calculus operations represented by Equation (1). (Some embodiments transmit the coefficients b from Equation (2) to a remote site for record storage and further analysis, since they effectively compress much of the information in the data window. If coefficients are to be transmitted, the [Mx(2N+1)] matrix $(A^T Ar)^{-1} A^T$ is pre-computed and then multiplied by each data window locally on the fly. Other embodiments omit such compression and/or transmission, or only do it locally on remote command, and thereby save some power and processing complexity.)

$$b = (A^T A)^{-1} A^T X , \qquad (2)$$

provided the inverse $(A^T A)^{-1}$ exists -- when the rows of the matrix A are linearly independent (full rank) and enough data points $N_W = (2N+1)$ deliver enough corresponding columns of the matrix sufficient for an inverse to be delivered.

In the special case of a polynomial transform process, a matrix of indices is raised to powers, wherein the jth column element $A_{nj}$ in the nth row of transform matrix A is raised to a power: $n^j$. In other words, for the 2N+1 different values of n from -N to +N in the window of a data stream X(i+n), the transform finds a set of coefficients $b_j$ for a well-fitting power series to approximate all the values. Such a power series in general is

$$X'(i+n) = b_o + b_1 n + b_2 n^2 + b_3 n^3 + b_4 n^4 + ... \ b_M n^M . \qquad (3)$$

Savitzky-Golay filtering outputs as the filter output g(i) for the window indexed i the value of $b_0$ estimated by Equation (2) for each data window, and successively window-by-window for successive indices g(i). Rows of matrix A are orthogonal when the inner product is zero for any pair of different ones of them. These rows are illustrated in Table 1, below. The rows of values $A_{nj}$ in matrix row n are nonorthogonal for the example of a polynomial transform. ("^" signifies raising to a power) .

Table 1: Arrangement of Matrix $A^T$

Power m (Order M)

| Points | 0 | 1 | 2 | 3 ... | M |
|---|---|---|---|---|---|
| n=-N: | [1 | (-N) | (-N)^2 | (-N)^3 ... | ...(-N)^M]. |
| ... | | | | | |
| n=-1: | [1 | -1 | 1 | -1 ... | (-1)^M] |
| n=0: | [1 | 0 | 0 | 0 ... | 0^M] |
| n=1: | [1 | 1 | 1 | 1 ... | 1^M] |
| ... | | | | | |
| n=+N: | [1 | N | N^2 | N^3 ... | N^M]. |

Next, the process finds an estimated data stream

$$X' = Ab = A (A^T A)^{-1} A^T X . \qquad (4)$$

An electronic process is set up in a processing circuit as represented by Equation (2) and electronically executed by the processing circuit. For Savitzky-Golay filtering, the process is optimized to only find g(i) as the estimated value

of $b_0$ and also to perform as much off-line pre-computation as possible. Accordingly, Equation (4) is revised as in Equation (5) below, to use only the n=0 row [1xM] of the first premultiplying matrix A instead of the whole matrix A in Equation (4),

$$g(i) = [1\ 0\ 0\ ...0]\ (A^TA)^{-1}\ A^T\ X(i)\ . \qquad (5)$$

Sometimes a mathematical presentation of Savitzky-Golay filtering regards the window as multiply-added by a set of (2N+1) filter coefficients c(n). Here, a [1x(2N+1)] filter coefficient vector C is introduced so that

$$g(i) = C\ X(i) = \sum_{n=-N}^{+N}\ c(n)x(i+n), \qquad (6)$$

where

$$C = [1\ 0\ 0\ ...0]\ (A^TA)^{-1}\ A^T\ . \qquad (7)$$

In Equation (8), an alternative notation CI equivalent to Equation (6) postmultiplies Equation (7) by a [(2N+1)x(2N+1)] identity matrix I and designates each of the columns of that identity matrix I as [(2N+1)x1] unit vectors $\varepsilon_n$. The phrase 'unit vector' for $\varepsilon_n$ means a [(2N+1)x1] vector of all zeroes except for a one (1) at the nth row position. Furthermore, only the matrix inversion computations to form the first row of inverse matrix $(A^TA)^{-1}$ are relevant and are performed, considering the pre-multiplication by [1xM] row n=0 vector [1 0 0...0]. Thus, the filter coefficients are also equivalently expressed in the notation of Equation (8), which is equivalent to Equation (7).

$$c(n) = \{(A^TA)^{-1}\ (A^T\ \varepsilon_n\ )\}_0\ . \qquad (8)$$

The Savitzky-Golay filter does a local polynomial fit in a least square sense. For a given input variable data window x(i+n) and window of length 2N+1 and chosen polynomial degree M, the filter output is given by g(i). Filter coefficients c(n) -- 2N+1 of them -- are computed, e.g. off-line, by electronic operations represented by Equation (7) or (8) and loaded into flash memory of a small signal processing unit either worn on the person or provided nearby and coupled wirelessly to the accelerometer sensor 10. The signal processing unit suitably has a digital signal processor circuit such as processor 220 that electronically performs multiply-accumulates (MACs) represented by Equation (6) according to a stored program accessing the filter coefficients c(n).

Some other embodiments use windows that are not centered around the value at index n used as the output (e.g., n=0). Other matrix types may be used instead of a polynomial transform matrix A (Table 1). The skilled worker chooses the desired transform, the window (frame) size (e.g., 2N+1) and the order M. Also, note that g(i) output of a first filter procedure produces a data stream that itself can be windowed as represented by column vector $g_1(i_2)$ in Equation (9B). Equations (9A), (9B) cascade two lower order filters of Equation (4) and the electronic processing complexity of the computations in implementation is then minimized. The transform matrices A1 and A2 can be the same or different, the window sizes $(2N_1+1)$ and $(2N_2+1)$ can be the same or different, and the orders $M_1$ and $M_2$ can be the same or different, independently of each other.

$$g_1(i_1) = [1\ 0\ 0\ ...0]\ (A_1^TA_1)^{-1}\ A_1^T\ X(i_1)\ ,\ \text{and} \qquad (9A)$$

$$g_2(i_2) = [1\ 0\ 0\ ...0]\ (A_2^TA_2)^{-1}\ A_2^T\ g_1(i_2). \qquad (9B)$$

Some embodiments may also apply to the SG process a diagonal weighting matrix W which is all zeroes in a [(2N+1)x(2N+1)] matrix except for weights down the main diagonal. The weights can, for instance, be one at the middle of the diagonal and diminish symmetrically in value farther from the middle of the diagonal. Equation (1) is replaced by:

$$\Delta b = - (\nabla \nabla \|W(X-Ab)\| )^{-1}\ \nabla\ \|W(X-Ab)\|\ . \qquad (10)$$

Then the electronic process represented by Equation (5) for the output instead is:

$$g(i) = [1\ 0\ 0\ ...0]\ (A^T W A)^{-1}\ A^T W\ X(i). \qquad (11)$$

The selection of transform type and matrix A is fixed/predefined by configuration or determined semi-static manner in some embodiments. Dynamic configuration or selection of the matrix A or transform type or parameters of a given transform is contemplated.

Some other embodiments store and average a set of values from the transform output of Equation (4) from different windowed segments of the data stream X. Thus, several filters are in parallel and averaged in an offset manner. All the values represent a reconstructed value for a same instant of time (i+n) = t. This approach not only uses the n=0 row to approximate $b_0$ but also uses the transform approximations to the other coefficients that are available from Equation (3). The electronic processor 220 (and/or 240) electronically performs Equation (12), where X'(i+n) is from Equation (4).

$$g(t) = [1/(2N_1+1)] \sum_{n=-N}^{+N} X'(i+n)|_{(i+n\,=\,t)} \ .\qquad (12)$$

Thus, other types of processes can accord with the teachings herein, whether Savitzky-Golay or not. The skilled worker sets up a test bench with library accelerometer-based waveforms and then makes the transform matrix choices, choice of number of points (2N+1), and choice of order-value M, either manually or by an automated process. Filtering choices are tested by visual inspection of a display of output from FIG. 1 process or by automated process according to metrics of false negatives and false positives, etc. Transform matrix A values, and values of (2N+1) and M, $2N_1+1$, etc., for one or more such filter processes having favorable metrics are then loaded into the monitoring device flash memory or hard drive and executed in real time on the processor. A transform for an embodiment approximates an actual data stream vector x(i+n) and produces an output signal stream that follows the heart sound peaks well over time in response to a data stream X herein derived from a body-worn accelerometer. Some embodiments have reduced processing complexity by using low enough frame size (2N+1), order M and/or using an efficient transform matrix A to achieve desired performance for the purposes for which the monitoring is intended. The same transform is desirably low-complexity and well-performing over numerous patients, accelerometers and their positioning on the body, and in different environments of use, such as clinic, hospital, home, exercise venue, etc.

In envelope-based noise rejection 150, an amplitude envelope is generated. The residue signal stream r(i) = x(i)-g(i) of of FIGS. 3 and 4A has some remaining noise from subtracting step 140 that subtracts the smoothing filter output g(i) from the LPFsupplied input x(i). An envelope is fitted to the residue. Amplitude-based processed output R(i) is derived from the envelope-fitted residue r(i). Noise n(i) near the horizontal axis is substantially reduced. Operations can use an envelope-related variable gain function, or generates zero signal output when envelope is below a low threshold that passes peaks.

In folded correlation 160, an input data stream of residue r(i) = x(i)-g(i), or envelope-processed residue R(i) comes in, and g(i) is the output of the smoothing filter such as by Equation (6) or from a buffer memory for it. R(i) is windowed by a further data window (a frame) of length $2N_2 +1$ (with points accessed by an index n=0,1,2... $N_2$).

The output $f_c(i)$ of the folded correlation is given by Equation (13):

$$f_c(i) = \sum_{n=0}^{N2} R(i - N_2 + n)\ R(i + N_2 - n) \ . \qquad (13)$$

The digital data stream for heart monitoring residue signal samples R(i) from the smoothing filter subtraction is successively processed in overlapping frames indexed i. The value of $2N_2 + 1$ approximates the width $t_W$ of a desired signal event (e.g., an S1, S2, or R-wave). S1 is perhaps about 100-150 milliseconds long. If decimated sampling frequency $f_S$ is 1000 Samples/sec, the value of $2N_2 + 1$ is established, e.g., as an odd number between 101 and 151, and N2 is some number between 50 and 75 inclusive. Thus,

$$N2 = RND(t_W\ f_S\ /2). \qquad (14)$$

The value of N2 can be configured in flash memory, and can be selected or altered by a local or remote operator using the FIG. 2 system. In the electronic folded correlation process 160 represented by Equation (13), the heart monitoring residue samples R(i + N2 - n) from the latter half of each frame are folded around the center heart monitoring sample R(i) in the frame and multiplied by dot product (sum of products in Eq. (13)) with heart monitoring residue samples R(i - N2 + n) in the earlier half of each frame. The result of the dot product is a folded correlation output signal stream $f_c(i)$ corresponding to instant i of the input residue signal stream R(i) in the center of the frame. Succeeding thresholding passes the S1 peaks and counts them. This concludes the Appendix.

Processing circuitry comprehends digital, analog and mixed signal (digital/analog) integrated circuits, ASIC circuits, PALs, PLAs, decoders, memories, and programmable and nonprogrammable processors, microcontrollers and other circuitry. Couplings and connections can be ohmic, capacitive, inductive, photonic, and direct or indirect via intervening circuits or otherwise as desirable. Flow diagrams and block diagrams are each interpretable as representing structure and/or process. However, the present invention is limited to a device as defined by the appended claims.

**Claims**

1.    An electronic monitoring device (600), comprising:

A triple axis accelerometer sensor (10,610) having an output;
an electronic processor (670) having an input coupled to the output of the sensor and an output; and
a memory (660) holding instructions for the electronic processor and coupled to the electronic processor so that the electronic processor is operable to isolate a cardiac signal including cardiac pulses combined with other cardiac signal variations, and the electronic processor (670) is further operable to execute a filter that separates a varying blood flow signal from the cardiac pulses, and to output information based on at least the varying blood flow signal,
wherein the electronic processor (670) is further operable to also separate cardiac pulses from the cardiac signal and to produce an electronic representation of a hemodynamic parameter based on a time difference between a selected cardiac pulse and a peak in the varying blood flow signal after the selected cardiac pulse.

2.    The device of claim 1, wherein the accelerometer sensor comprises a chest accelerometer sensor circuit (610), and wherein the electronic processor is further operable to provide a hemodynamic parameter, a heart rate, and a respiration parameter, all based on the chest accelerometer sensor circuit.

3.    The device of claim 2, wherein the filter for the blood flow signal further smoothes the cardiac signal using a low-order polynomial filter with a window size of approximately 200 milliseconds.

4.    The device of claim 1, wherein the electronic processor (670) is further operable to perform electronic peak detection on the varying blood flow signal to identify a flow peak amplitude and a time location of that peak.

5.    The device of claim 4, wherein the electronic processor (670) is further operable to output an electronic representation of iso-volumic contraction interval using the varying blood flow signal.

6.    The device of claim 5, wherein the electronic processor (670) is further operable to produce an electronic representation of contractility using the iso-volumic contraction interval.

7.    The device of claim 6, wherein the electronic processor (670) is further operable to perform electronic peak detection on the varying blood flow signal to identify a flow peak amplitude (PAmp) and to generate a hemodynamic parameter as a function of the flow peak amplitude (PAmp).

8.    The device of claim 7, wherein the electronic processor (670) is further operable to derive a signal, related to at least one of respiration, intrapleural pressure, and intrathoracic pressure, from variations in beat-by-beat values of flow peak amplitude (PAmp) of the varying blood flow signal.

9.    The device of claim 1 or claim 8, wherein the electronic processor (670) is further operable to obtain cardiac pulses and a heart rate (HR) from the varying blood flow signal, and further to generate a cardiac output as a function of flow peak amplitude (PAmp) of the varying blood flow signal times the heart rate (HR).

10.    The device of claim 1, wherein the accelerometer sensor (10,610) has a broadside portion and an accelerometer sensor circuit having perpendicular axes of acceleration sensitivity parallel to the broadside portion; and wherein the electronic processor (670) is operable to combine signals from the accelerometer sensor circuit representing at least both of those axes of acceleration sensitivity.

**11.** The device of claim 10, wherein the electronic processor (670) is further operable to produce a measurement of jitter across heart beats of a time difference between a selected cardiac pulse and a peak in the varying blood flow signal after the selected cardiac pulse.

**12.** The device of claim 1, wherein the electronic processor (670) is further operable to perform electronic peak detection on the varying blood flow signal to identify a flow peak amplitude (PAmp) and to generate a hemodynamic parameter as a function of the flow peak amplitude (PAmp).

**Patentansprüche**

**1.** Elektronische Überwachungsvorrichtung (600), umfassend:

einen Dreiachsen-Beschleunigungsmesssensor (10, 610) mit einem Ausgang;

einen elektronischen Prozessor (670) mit einem Eingang, der mit dem Ausgang des Sensors gekoppelt ist, und einem Ausgang; und
einen Speicher (660), der Anweisungen für den elektronischen Prozessor vorhält und mit dem elektronischen Prozessor gekoppelt ist, so dass der elektronische Prozessor dazu funktionsfähig ist, ein Herzsignal einschließlich Herzimpulsen kombiniert mit anderen Herzsignalschwankungen zu isolieren, und der elektronische Prozessor (670) ferner dazu funktionsfähig ist, ein Filter auszuführen, das ein sich änderndes Blutflusssignal von den Herzimpulsen trennt, und Informationen auf der Grundlage mindestens des sich ändernden Blutflusssignals auszugeben,

wobei der elektronische Prozessor (670) ferner dazu funktionsfähig ist, auch die Herzimpulse vom Herzsignal zu trennen und eine elektronische Darstellung eines hämodynamischen Parameters auf der Grundlage einer Zeitdifferenz zwischen einem ausgewählten Herzimpuls und einer Spitze in dem sich ändernden Blutflusssignal nach dem ausgewählten Herzimpuls zu erzeugen.

**2.** Vorrichtung nach Anspruch 1, wobei der Beschleunigungsmesssensor einen Thorax-Beschleunigungsmesssensor-Schaltkreis (610) umfasst und wobei der elektronische Prozessor ferner dazu funktionsfähig ist, einen hämodynamischen Parameter, eine Herzfrequenz und einen Atmungsparameter bereitzustellen, die alle auf dem Thorax-Beschleunigungsmesssensor-Schaltkreis beruhen.

**3.** Vorrichtung nach Anspruch 2, wobei das Filter für das Blutflusssignal ferner das Herzsignal mithilfe eines Polynomfilters niedriger Ordnung mit einer Fenstergröße von ca. 200 Millisekunden glättet.

**4.** Vorrichtung nach Anspruch 1, wobei der elektronische Prozessor (670) ferner dazu funktionsfähig ist, eine elektronische Spitzenerkennung an dem sich ändernden Blutflusssignal durchzuführen, um eine Flussspitzenamplitude und einen Zeitpunkt dieser Spitze zu erkennen.

**5.** Vorrichtung nach Anspruch 4, wobei der elektronische Prozessor (670) ferner dazu funktionsfähig ist, eine elektronische Darstellung des Intervalls der isovolumetrischen Kontraktionen mithilfe des sich ändernden Blutflusssignals auszugeben.

**6.** Vorrichtung nach Anspruch 5, wobei der elektronische Prozessor (670) ferner dazu funktionsfähig ist, eine elektronische Darstellung der Kontraktionsfähigkeit mithilfe des Intervalls der isovolumetrischen Kontraktionen zu erzeugen.

**7.** Vorrichtung nach Anspruch 6, wobei der elektronische Prozessor (670) ferner dazu funktionsfähig ist, eine elektronische Spitzenerkennung an dem sich ändernden Blutflusssignal durchzuführen, um eine Flussspitzenamplitude (PAmp) zu erkennen und einen hämodynamischen Parameter als Funktion der Flussspitzenamplitude (PAmp) zu erzeugen.

**8.** Vorrichtung nach Anspruch 7, wobei der elektronische Prozessor (670) ferner dazu funktionsfähig ist, ein Signal, das mit der Atmung, dem intrapleuralen Druck und/oder dem intrathorakalen Druck in Zusammenhang steht, aus den Schwankungen der Schlag-zu-Schlag-Werte der Flussspitzenamplitude (PAmp) des sich ändernden Blutflusssignals abzuleiten.

**9.** Vorrichtung nach Anspruch 1 oder Anspruch 8, wobei der elektronische Prozessor (670) ferner dazu funktionsfähig ist, Herzimpulse und eine Herzfrequenz (HR) aus dem sich ändernden Blutflusssignal zu erhalten und ferner einen Herzausgang als Funktion der Flussspitzenamplitude (PAmp) des sich ändernden Blutflusssignals mal der Herzfrequenz (HR) zu erzeugen.

**10.** Vorrichtung nach Anspruch 1, wobei der Beschleunigungsmesssensor (10, 610) einen Breitseitenabschnitt und einen Beschleunigungsmesssensor-Schaltkreis mit senkrechten beschleunigungsempfindlichen Achsen parallel zum Breitseitenabschnitt aufweist; und wobei der elektronische Prozessor (670) dazu funktionsfähig ist, Signale vom Beschleunigungsmesssensor-Schaltkreis, die wenigstens beide dieser beschleunigungsempfindlichen Achsen darstellen, zu kombinieren.

**11.** Vorrichtung nach Anspruch 10, wobei der elektronische Prozessor (670) ferner dazu funktionsfähig ist, eine Messung des Jitters über Herzschläge von einer Zeitdifferenz zwischen einem ausgewählten Herzimpuls und einer Spitze in dem sich ändernden Blutflusssignal nach dem ausgewählten Herzimpuls zu erzeugen.

**12.** Vorrichtung nach Anspruch 1, wobei der elektronische Prozessor (670) ferner dazu funktionsfähig ist, eine elektronische Spitzenerkennung an dem sich ändernden Blutflusssignal durchzuführen, um eine Flussspitzenamplitude (PAmp) zu erkennen und einen hämodynamischen Parameter als Funktion der Flussspitzenamplitude (PAmp) zu erzeugen.

**Revendications**

**1.** Dispositif de surveillance électronique (600), comprenant :

un capteur d'accéléromètre à trois axes (10, 610) ayant une sortie ;
un processeur électronique (670) ayant une entrée couplée à la sortie du capteur et une sortie ; et
une mémoire (660) contenant des instructions pour le processeur électronique, et couplée au processeur électronique de sorte que le processeur électronique est opérationnel pour isoler un signal cardiaque comprenant des impulsions cardiaques combinées avec d'autres variations de signal cardiaque, et le processeur électronique (670) est en outre opérationnel pour exécuter un filtrage qui sépare un signal de débit sanguin variable des impulsions cardiaques, et pour transmettre des informations sur la base d'au moins le signal de débit sanguin variable,
le processeur électronique (670) étant en outre opérationnel pour séparer également les impulsions cardiaques du signal cardiaque et pour produire une représentation électronique d'un paramètre hémodynamique basé sur une différence de temps entre une impulsion cardiaque sélectionnée et un pic dans le signal de débit sanguin variable après l'impulsion cardiaque sélectionnée.

**2.** Dispositif selon la revendication 1, le capteur d'accéléromètre comprenant un circuit de capteur d'accéléromètre de poitrine (610),
et le processeur électronique étant en outre opérationnel pour fournir un paramètre hémodynamique, une fréquence cardiaque et un paramètre de respiration, tous basés sur le circuit de capteur d'accéléromètre de poitrine.

**3.** Dispositif selon la revendication 2, le filtre pour le signal de débit sanguin lissant en outre le signal cardiaque en utilisant un filtre polynomial d'ordre inférieur avec une taille de fenêtre d'environ 200 millisecondes.

**4.** Dispositif selon la revendication 1, le processeur électronique (670) étant en outre opérationnel pour réaliser une détection de pic électronique sur le signal de débit sanguin variable pour identifier une amplitude de pic de débit et un emplacement temporel de ce pic.

**5.** Dispositif selon la revendication 4, le processeur électronique (670) étant en outre opérationnel pour émettre une représentation électronique de l'intervalle de contraction iso-volumique en utilisant le signal de débit sanguin variable.

**6.** Dispositif selon la revendication 5, le processeur électronique (670) étant en outre opérationnel pour produire une représentation électronique de la contractilité en utilisant l'intervalle de contraction iso-volumique.

**7.** Dispositif selon la revendication 6, le processeur électronique (670) étant en outre opérationnel pour réaliser une détection de pic électronique sur le signal de débit sanguin variable pour identifier une amplitude de pic de débit (PAmp) et pour générer un paramètre hémodynamique en fonction de l'amplitude de pic de débit (PAmp).

8. Dispositif selon la revendication 7, le processeur électronique (670) étant en outre opérationnel pour dériver un signal, lié à au moins l'un parmi la respiration, la pression intrapleurale et la pression intrathoracique, à partir des variations des valeurs battement par battement de l'amplitude du pic de débit (PAmp) du signal de débit sanguin variable.

9. Dispositif selon la revendication 1 ou selon la revendication 8, le processeur électronique (670) étant en outre opérationnel pour obtenir des impulsions cardiaques et une fréquence cardiaque (HR) à partir du signal de débit sanguin variable, et pour générer en outre une sortie cardiaque en fonction de l'amplitude de pic de débit (PAmp) du signal de débit sanguin variable multiplié par la fréquence cardiaque (HR).

10. Dispositif selon la revendication 1, le capteur d'accéléromètre (10, 610) ayant une partie de côté large et un circuit de capteur d'accéléromètre ayant des axes perpendiculaires de sensibilité d'accélération parallèles à la partie de côté large ; et le processeur électronique (670) étant opérationnel pour combiner des signaux provenant du circuit de capteur d'accéléromètre représentant au moins les deux de ces axes de sensibilité d'accélération.

11. Dispositif selon la revendication 10, le processeur électronique (670) étant en outre opérationnel pour produire une mesure de gigue à travers les battements cardiaques d'une différence de temps entre une impulsion cardiaque sélectionnée et un pic dans le signal de débit sanguin variable après l'impulsion cardiaque sélectionnée.

12. Dispositif selon la revendication 1, le processeur électronique (670) étant en outre opérationnel pour réaliser une détection de pic électronique sur le signal de débit sanguin variable pour identifier une amplitude de pic de débit (PAmp) et pour générer un paramètre hémodynamique en fonction de l'amplitude de pic de débit (PAmp).

*FIG. 1*

FIG. 2

600

690 — TELE-MEDICINE THERAPEUTIC/ ASSISTIVE DEVICE

610 — CHEST ACCELEROMETER, CIRCUIT, BT MODEM

PATIENT

640.1 — WIRELESS TRANSMITTER/ RECEIVER MODEM

670 — WIRELESS RECEIVER/ TRANSMITTER MODEM AND PROCESSOR

650.1 — DISPLAY UNIT (HEART SOUND SIGNAL OR HEART RATE, AND HEMODYNAMICS)

660.1 — STORAGE UNIT

650.2 — DISPLAY UNIT (MOTION SIGNAL)

660.2 — STORAGE UNIT

650.3 — DISPLAY UNIT (RESPIRATION SIGNAL)

660.3 — STORAGE UNIT

STREAMING DATA AND CONTROL INTERFACE

DATA ACQUISITION/ SIGNAL PROCESSING

SHORT RANGE WIRELESS MODEM

622   624   620

640.2 — WIRELESS TRANSMITTER/ RECEIVER MODEM

STORAGE UNIT

630

640.3 — WIRELESS TRANSMITTER/ RECEIVER MODEM

680 — ALERT TO PROFESSIONAL, TO PATIENT, CAREGIVER AND/OR FAMILY MEMBER

EP 2 501 278 B1

*FIG. 3*

LPF/ADC INPUT1 (Z-AXIS)

110 — LOW PASS FILTER THE INPUT
(FILTER CUTOFF=50 Hz)

120 — DECIMATE BY 10

130 — SAVITZKY-GOLAY
POLYNOMIAL FILTERING
28th ORDER, 401 POINTS

BASELINE
WANDER

140 — SUBTRACT SAVITZKY-GOLAY
OUTPUT FROM THE LOW-PASS
FILTER OUTPUT TO OBTAIN HEART
SOUNDS, S1 AND S2

160 — PERFORM FOLDED CORRELATION
ON HEART SOUND SIGNAL

170 — PERFORM PEAK DETECTION

180 — CALCULATE HEART RATE

*FIG. 4A*

LPF/ADC INPUT2 (Y-AXIS)

910 — LOW PASS FILTER THE INPUT
(FILTER CUTOFF=50 Hz)

920 — DECIMATE BY 10

SAVITZKY-GOLAY POLYNOMIAL
FILTERING 4th ORDER — 930

940 — SUBTRACT SAVITZKY-GOLAY
OUTPUT FROM THE LOW-PASS
FILTER OUTPUT TO OBTAIN
RESIDUE INCLUDING HEART
SOUNDS, S1 AND S2

RINGY SIGNAL
INTERPRETED AS
BLOOD FLOW — 950

970 — PEAK DETECTION

960

980 — CALCULATE HEART RATE

RECOVER
FORCING
FUNCTION

ESTIMATE 2nd
ORDER SYSTEM
PARAMETERS — 965

*FIG. 4B*

EP 2 501 278 B1

*FIG. 5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5935081 A
- US 2004215264 A1
- JP 2004305268 A